## Europäisches Patentamt
## European Patent Office
## Office européen des brevets

(11) Veröffentlichungsnummer: **0 174 563**
**B1**

(12) # EUROPÄISCHE PATENTSCHRIFT

(45) Veröffentlichungstag der Patentschrift:
25.11.87

(51) Int. Cl.⁴: **C 07 C 87/66**, C 07 C 85/11

(21) Anmeldenummer: 85110919.9

(22) Anmeldetag: 30.08.85

(54) Verfahren zur Herstellung von 1-Naphthylamin.

(30) Priorität: 11.09.84 DE 3433240

(43) Veröffentlichungstag der Anmeldung:
19.03.86 Patentblatt 86/12

(45) Bekanntmachung des Hinweises auf die Patenterteilung:
25.11.87 Patentblatt 87/48

(84) Benannte Vertragsstaaten:
CH DE FR GB IT LI SE

(56) Entgegenhaltungen:
DE-A-2 452 015
DE-A-2 456 308
DE-A-2 519 838
DE-B-2 036 313
GB-A-227 481
US-A-3 499 034

(73) Patentinhaber: BAYER AG, Konzernverwaltung RP Patentabteilung, D-5090 Leverkusen 1 Bayerwerk (DE)

(72) Erfinder: Zander, Jürgen, Dr., Tannenweg 5, D-5090 Leverkusen 3 (DE)
Erfinder: Kappler, Ulrich, Dr., Rheindorfer Strasse 163c, D-4018 Langenfeld (DE)

LIBER, STOCKHOLM 1987

EP 0 174 563 B1

### Beschreibung

Die Erfindung betrifft ein Verfahren zur Herstellung von 1-Naphthylamin durch katalytische Hydrierung von 1-Nitronaphthalin bei erhöhter Temperatur und erhöhtem Druck in Gegenwart von inerten, mit Wasser mischbaren organischen Lösungsmitteln.

Die Herstellung von 1-Naphthylamin wird üblicherweise im technischen Maßstab durch Reduktion von 1-Nitronaphthalin mit Eisen in verdünnter Salzsäure (Béchamp-Reduktion) oder katalytisch mit Wasserstoff in Gegenwart von Nickel-Katalysatoren (z. B. Raney-Nickel) oder durch Umsetzung von 1-Naphthol mit Ammoniak und Ammoniumbisulfit (Bucherer-Reaktion) durchgeführt (vgl. z. B. Kirk-Othmer, Band 4, 1. Ausgabe, Seite 258 bis 260). So beträgt beispielsweise die Ausbeute bei der Reduktion mit Eisen etwa 90 % der Theorie (siehe Kirk-Othmer, Band 4, Seite 260); bei der katalytischen Hydrierung mit Nickel-Katalysatoren werden Ausbeuten von über 90 %, z. B. 96,4 %, angegeben (vgl. US-PS 21 05 321, J. Chem. Soc. Japan, 54, 371 bis 373 (1951) und C.A. 48, 1979d).

Nachteilig bei den katalytischen Reduktionsverfahren ist, daß es nicht gelingt, diese Verfahren auch auf die Reduktion von gereinigtem 1-Nitronaphthalin, das praktisch frei von Schwefelverbindungen und isomerem 2-Nitronaphthalin ist, zu übertragen. Wie eigene Versuche mit gereinigtem 1-Nitronaphthalin zeigten, entsteht bei der katalytischen Hydrierung mit Raney-Nickel unter den üblichen Reaktionsbedingungen nur in untergeordnetem Maße das gewünschte Naphthylamin. Der Angriff des Wasserstoffs erfolgt gleichzeitig und überwiegend am aromatischen Kern und es werden hauptsächlich die nicht gewünschten kernhydrierten Aminoverbindungen erhalten (vgl. Beispiel 2 im Beispielteil).

Weiterhin ist beschrieben, Nitronaphthaline bei erhöhter Temperatur und erhöhtem Druck in Gegenwart von z. B. Edelmetallkatalysatoren, die gegebenenfalls auf Träger aufgebracht sein können, zu hydrieren (vgl. z. B. DE-OSen 24 56 308 und 25 19 838). Die in den genannten deutschen Offenlegungsschriften beschriebenen Hydrierungen von Nitroverbindungen erfordern jedoch spezielle Bedingungen, die einige Nachteile für ein großtechnisches Verfahren mit sich bringen. So wird die Hydrierung gemäß der DE-OS 24 56 308 unter Zusatz von speziellen Verdünnungsmitteln durchgeführt, in denen die zu hydrierende Nitroverbindung löslich ist und das im wesentlichen aus Aminoverbindung und Wasser bestehende Hydrier-Produkt praktisch unlöslich ist. Das in der DE-OS 25 19 838 beschriebene Verfahren erfolgt in der Rieselphase an fest angeordneten Katalysatoren. Nachteilig bei dem Verfahren der DE-OS 24 56 308 ist der Zusatz spezieller Verdünnungsmittel, die hohe Verdünnung der Reaktionspartner mit daraus resultierenden geringen Durchsätzen sowie die technisch aufwendige Rückgewinnung der Lösungsmittel, was zu Lasten der Wirtschaftlichkeit des Verfahrens geht.

Bei dem Verfahren der DE-OS 25 19 838 ist die Rieselphase wenig vorteilhaft, da ebenfalls in hoher Verdünnung gearbeitet wird, wodurch nur geringe Durchsätze möglich sind, was ebenfalls zu Lasten der Wirtschaftlichkeit des Verfahrens geht. Außerdem sind Ablagerungen auf dem Festbettkontakt zu erwarten, was zu hohen Druckverlusten in der Anlage, gegebenenfalls sogar zu Verstopfungen, führen kann.

Es wurde nun ein Verfahren zur Herstellung von 1-Naphthylamin durch katalytische Hydrierung von 1-Nitronaphthalin bei erhöhter Temperatur und erhöhtem Druck in Gegenwart von inerten, mit Wasser mischbaren organischen Lösungsmitteln gefunden, das dadurch gekennzeichnet ist, daß man gereinigtes 1-Nitronaphthalin, das praktisch frei von Schwefelverbindungen und isomerem 2-Nitronaphthalin ist, bei Temperaturen von 150 bis 250°C und Drücken von 50 bis 300 bar in Gegenwart von Platin/Aktivkohle hydriert, wobei die mittlere Verweilzeit des Reaktionsgemisches im Reaktor 3 bis 30 Minuten beträgt.

Erfindungsgemäß wird gereinigtes 1-Nitronaphthalin in das Verfahren eingesetzt, das praktisch frei von Schwefelverbindungen, wie Nitrobenzothiophenen, sowie frei von dem isomeren 2-Nitronaphthalin ist.

Üblicherweise enthält das gereinigte 1-Nitronaphthalin weniger als 10 ppm Schwefel, bevorzugt 3 bis 5 ppm Schwefel in Form von Schwefelverbindungen und weniger als 10 ppm, bevorzugt 1 bis 3 ppm an 2-Nitronaphthalin.

Die Hydrierung des gereinigten 1-Nitronaphthalins wird bevorzugt bei Temperaturen von 160 bis 200°C und bei Drücken ($H_2$) von 80 bis 150 bar durchgeführt.

Als Hydrierkatalysator wird in das erfindungsgemäße Verfahren ein Platin/Aktivkohle-Katalysator eingesetzt, bei dem das Platin in Mengen von 0,3 bis 7, bevorzugt 0,5 bis 2, Gew.-%, bezogen auf den fertigen Katalysator, auf den Aktivkohleträger aufgebracht ist. Die Herstellung des Hydrierkatalysators erfolgt in bekannter Weise durch Tränken oder Besprühen des Aktivkohleträgers mit einer wäßrigen Platinsalzlösung, z. B. einer Hexachloroplatinsäure-Lösung ($H_2PtCl_6$). Anschließend wird die auf den Träger aufgebrachte Platinverbindung in üblicher Weise, z. B. mit Formaldehyd und Hydrazin in alkalischer Lösung oder mit Wasserstoff, zum Metall reduziert.

Im Kreislauf des kontinuierlichen Prozesses befinden sich 1 bis 2 Gew.-% des Pt/Aktivkohle-Katalysators bezogen auf den Flüssigkeitsinhalt der Hydrieranlage. Die Menge des eingesetzten Frischkatalysators beträgt im allgemeinen etwa 2 bis 20 g, bevorzugt 4 bis 8 g, bezogen auf 1 k Mol des eingesetzten 1-Nitronaphthalins.

Erfindungsgemäß wird die Reduktion des gereinigten 1-Nitronaphthalins in Gegenwart von inerten, mit Wasser mischbaren organischen Lösungsmitteln, wie niedere aliphatische und/oder cycloaliphatische Alkohole, durchgeführt. Als niedere Alkohole seien z. B. genannt: Methanol, Ethanol, n-Propanol, Isopropanol, n-Butanol, iso-Butanol und/oder tert.-Butanol, bevorzugt Isopropanol.

Die Menge des eingesetzten organischen Lösungsmittels ist nicht kritisch und kann leicht durch Vorversuche ermittelt werden. Üblicherweise setzt man etwa 1 bis 5 Gew.-Teile, bevorzugt 1,5 bis 3 Gew.-Teile Lösungsmittel, bezogen auf 1 Gew.-Teil 1-Nitronaphthalin ein.

Das erfindungsgemäße Verfahren wird so durchgeführt, daß die mittlere Verweilzeit des Reaktionsgemisches im Reaktor 3 bis 30, bevorzugt 5 bis 20, besonders bevorzugt 8 bis 12 Minuten beträgt.

Das erfindungsgemäße Verfahren kann sowohl diskontinuierlich als auch kontinuierlich durchgeführt werden. Bevorzugt wird es kontinuierlich durchgeführt.

Nach dem erfindungsgemäßen Verfahren wird das 1-Nitronaphthalin in hoher Reinheit ($\geq$ 99,5 %) und in hohen Ausbeuten (> 99 %) erhalten.

Das erfindungsgemäße Verfahren gestaltet sich auch deshalb technisch so vorteilhaft, weil keine speziellen Lösungsmittelsysteme verwendet werden (vgl. hierzu DE-OS 24 56 308), die u. a. in technisch aufwendiger Weise aufgearbeitet werden müssen, und keine speziellen, z. B. mit bestimmten Zusätzen (Metalldotierungen) versehene Katalysatoren oder partiell vergiftete Katalysatoren eingesetzt werden, die wegen der hohen Herstellungskosten zu Lasten der Wirtschaftlichkeit des Verfahrens gehen.

Weiterhin überraschend ist besonders, daß nicht alle Edelmetallkatalysatoren für das erfindungsgemäße Verfahren gleich gut geeignet sind, sondern nur der vorstehend beschriebene Platin auf Aktivkohle-Katalysator (vgl. hierzu das Vergleichsbeispiel 3 im Beispielteil).

## Beispiel 1

In die Reaktoren eines aus zwei Haupt- und einem Nachreaktor mit je 450 Liter Inhalt bestehenden Systems werden pro Stunde kontinuierlich 2000 kg an gereinigtem 1-Nitronaphthalin, das einen Gehalt an Schwefelverbindungen von umgerechnet 3 ppm Schwefel und einen Gehalt an 2-Nitronaphthalin von 3 ppm hat, 4200 kg Isopropanol/Wassergemisch (Wassergehalt etwa 15 Gew.-%) und 3000 kg Katalysator-haltige Fertigproduktlösung eingepumpt. Im Kreislauf befinden sich 1 bis 2 Gew.-% Katalysator (1 % Platin auf Aktivkohle). Stündlich wird ca. 0,05 bis 0,1 kg verbrauchter Katalysator ersetzt. Der Wasserstoffdruck wird auf 100 bar und die Temperatur durch Kühlwasser auf 160 bis 195°C gehalten. Nach einer Verweilzeit von etwa 8 Minuten wird das Produkt, das völlig umgesetzt und praktisch frei von Nitroverbindungen ist, aus dem Reaktor abgezogen.

Der pH-Wert der die Diaminverbindung enthaltende Lösung beträgt etwa 9 bis 12. Das durch Destillation wiedergewonnene Isopropanol/Wassergemisch kann ohne weitere Reinigung direkt wieder in die Reaktion eingesetzt werden. Das von Wasser weitgehend befreite rohe, nahezu farblose 1-Naphthylamin hat einen Erstarrungspunkt von 48,4°C; die Ausbeute beträgt 99,5 % der Theorie; die Reinheit des Produktes liegt bei 99,7 %.

## Beispiel 2

In der obengenannten Apparatur wurden unter den gleichen Bedingungen wie in Beispiel 1 beschrieben, pro Stunde 2000 kg gereinigtes 1-Nitronaphthalin in Gegenwart von Raney-Nickel (dotiert mit 15 % Eisen) hydriert (Katalysatorkonzentration im Kreis ca. 3 %). Es entstanden in nur 48,1 % Ausbeute 1-Naphthylamin und in 51,9 % Ausbeute verschiedene partiell und vollständig kernhydrierte Aminoverbindungen (Aminodekalin und Aminotetraline).

## Beispiel 3

Unter vergleichbaren Bedingungen wie unter Beispiel 1 beschrieben, wurde 1-Nitronaphthalin in Gegenwart von 1 % Palladium auf Aktivkohle hydriert. Es entstand in quantitativer Ausbeute ein Gemisch, das nur zu 90 % aus 1-Naphthylamin und zu 10 % aus kernhydrierten Aminoverbindungen bestand.

## Patentansprüche

1. Verfahren zur Herstellung von 1-Naphthylamin durch katalytische Hydrierung von 1-Nitronaphthalin bei erhöhter Temperatur und erhöhtem Druck in Gegenwart von inerten, mit Wasser mischbaren organischen Lösungsmitteln, dadurch gekennzeichnet, daß man gereinigtes 1-Nitronaphthalin, das praktisch frei von Schwefelverbindungen und isomerem 2-Nitronaphthalin ist, bei Temperaturen von 150 bis 250°C und Drücken von 50 bis 300 bar in Gegenwart von Platin/Aktivkohle hydriert, wobei die mittlere Verweilzeit des Reaktionsgemisches im Reaktor 3 bis 30 Minuten beträgt.

2. Verfahren nach Anspruch 1, dadurch gekennzeichnet, daß das eingesetzte 1-Nitronaphthalin weniger als 10 ppm Schwefel in Form von Schwefelverbindungen enthält.

3. Verfahren nach Ansprüchen 1 und 2, dadurch gekennzeichnet, daß das eingesetzte 1-Nitronaphthalin 3 bis 5 ppm Schwefel in Form von Schwefelverbindungen enthält.

4. Verfahren nach Ansprüchen 1 bis 3, dadurch gekennzeichnet, daß das eingesetzte 1-Nitronaphthalin weniger als 10 ppm 2-Nitronaphthalin enthält.

5. Verfahren nach Ansprüchen 1 bis 4, dadurch gekennzeichnet, daß das eingesetzte 1-Nitronaphthalin 1 bis 3 ppm 2-Nitronaphthalin enthält.

6. Verfahren nach Ansprüchen 1 bis 5, dadurch gekennzeichnet, daß man die Umsetzung bei Temperaturen von 160 bis 200°C durchführt.

7. Verfahren nach Ansprüchen 1 bis 6, dadurch gekennzeichnet, daß man die Umsetzung bei Drücken von 80 bis 150 bar durchführt.

8. Verfahren nach Ansprüchen 1 bis 7, dadurch gekennzeichnet, daß die mittlere Verweilzeit des Reaktionsgemisches im Reaktor 5 bis 20 Minuten beträgt.


**Claims**

1. Process for the preparation of 1-naphthylamine by the catalytic hydrogenation of 1-nitronaphthalene at elevated temperature and elevated pressure, in the presence of inert organic solvents miscible with water, characterized in that purified 1-nitronaphthalene, which is virtually free of sulphur compounds and isomeric 2-nitronaphthalene is hydrogenated at temperatures of 150 to 250°C and pressures of 50 to 300 bar, in the presence of platinum/activated charcoal, the average residence time of the reaction mixture in the reactor being 3 to 30 minutes.

2. Process according to Claim 1, characterized in that the 1-nitronaphthalene used contains less than 10 ppm of sulphur in the form of sulphur compounds.

3. Process according to Claims 1 and 2, characterized in that the 1-nitronaphthalene used contains 3 to 5 ppm of sulphur in the form of sulphur compounds.

4. Process according to Claims 1 to 3, characterized in that the 1-nitronaphthalene used contains less than 10 ppm of 2-nitronaphthalene.

5. Process according to Claims 1 to 4, characterized in that the 1-nitronaphthalene used contains 1 to 3 ppm of 2-nitronaphthalene.

6. Process according to Claims 1 to 5, characterized in that the reaction is carried out at temperatures of 160 to 200°C.

7. Process according to Claims 1 to 6, characterized in that the reaction is carried out at pressures of 80 to 150 bar.

8. Process according to Claims 1 to 7, characterized in that the average residence time of the reaction mixture in the reactor is 5 to 20 minutes.


**Revendications**

1. Procédé pour la fabrication de 1-naphtylamine par hydrogénation catalytique du 1-nitronaphtalène à température et sous pression élevées en présence de solvants organiques inertes miscibles avec l'eau, caractérisé en ce que l'on hydrogène un 1-nitronaphtalène purifié, qui est pratiquement exempt de composés soufrés et du 2-nitronaphtalène isomère, à des températures de 150 à 250°C et sous les pressions de 50 à 300 bar en présence de platine/charbon actif, selon lequel la durée moyenne de passage du mélange de réaction dans le réacteur est de 3 à 30 min.

2. Procédé selon la revendication 1, caractérisé en ce que le 1-nitronaphtalène utilisé contient moins de 10 ppm de soufre sous forme de composés soufrés.

3. Procédé selon les revendications 1 et 2, caractérisé en ce que le 1-nitronaphtalène utilisé contient 3 à 5 ppm de soufre sous forme de composés soufrés.

4. Procédé selon les revendications 1 à 3, caractérisé en ce que le 1-nitronaphtalène utilisé contient moins de 10 ppm de 2-nitronaphtalène.

5. Procédé selon les revendications 1 à 4, caractérisé en ce que le 1-nitronaphtalène utilisé contient 1 à 3 ppm de 2-nitronaphtalène.

6. Procédé selon les revendications 1 à 5, caractérisé en ce que l'on met en oeuvre la réaction à des températures de 160 à 200°C.

7. Procédé selon les revendications 1 à 6, caractérisé en ce que l'on met en oeuvre la réaction sous des pressions de 80 à 150 bar.

8. Procédé selon les revendications 1 à 7, caractérisé en ce que la durée moyenne de passage du mélange de réaction dans le réacteur est de 5 à 20 min.